# EUROPEAN PATENT APPLICATION

(11) **EP 3 915 967 A1**
(43) Date of publication of application: **01.12.2021**
(21) Application number: 21178869.0
(22) Date of filing: 22.06.2011
(51) Int. Cl.: C07C 17/357, C07C 21/18, C07C 17/354, C07C 19/08

(54) **PROCESS FOR THE MANUFACTURE OF FLUORINATED OLEFINS**

(30) Priority: 24.06.2010 US 82236510
(62) Divisional of application: 17170975.1
(71) Applicant: Honeywell International Inc., Morris Plains, NJ 07950 (US)
(72) Inventor: WANG, Haiyou, Morristown, NJ 07962 (US); TUNG, Hsueh Sung, Morristown, NJ 07962 (US)
(74) Representative: Stepney, Gregory John

(57) **Abstract**

A method of converting a first reaction stream comprising at least one pentafluoropropene to a final product stream comprising at least one compound of formula (II):

CHX=CZCF3 (II)

wherein X and Z are each independently H or F, with the proviso that X and Z are not the same, comprising processing said first reaction stream under conditions effective to convert said at least one pentafluoropropene to at least one compound of formula (II);
wherein said processing step comprises:
(a) reacting said at least one pentafluoropropene to obtain a first product stream comprising at least one pentafluoropropane and at least one compound of formula (I):

CH2XCHZCF3 (I)

wherein X and Z are each independently H or F, with the proviso that X and Z are not the same;
(b) separating said at least one compound of formula (I) from said first product stream to obtain a separated product stream and a second reaction stream, wherein said separated product stream comprises a higher weight percent of said at least one compound of formula (I) than said second reaction stream; and
(c) reacting said second reaction stream under conditions effective to produce said at least one compound of formula (II).

## Description

### CROSS-REFERENCE TO RELATED APPLICATIONS

This application claims priority under 35 U.S.C. § 119(e) to U.S. Application No. 12/822,365, filed on June 24, 2010, the disclosure of which is incorporated herein by reference in its entirety.

### BACKGROUND OF THE INVENTION

### 1. Field of Invention:

The present invention relates to novel methods of preparing fluorinated organic compounds, and particularly to methods of producing fluorinated olefins.

### 2. Description of Related Art:

Hydrofluorocarbons ("HFCs"), in particular hydrofluoro-olefins ("HFOs") such as tetrafluoropropenes (including 2,3,3,3-tetrafluoropropene ("HFO-1234yf") and 1,3,3,3-tetrafluoropropene ("HFO-1234ze")) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons ("CFCs") and hydrochlorofluorocarbons ("HCFCs"), both of which potentially damage the Earth's ozone layer, HFCs do not contain chlorine and thus pose no threat to the ozone layer.

Several methods for preparing fluorinated olefins from fluorinated alkanes are known. For example, U.S. Patent No. 7,560,602 discloses that CF₃CF=CHF ("HFO-1225ye") and HFO-1234yf can be produced via gas phase dehydrofluorination of CF₃CHFCHF₂ and CF₃CHFCH₂F, respectively, over a dehydrofluorination catalyst selected from the group consisting of one or more fluorinated metal oxides, metal fluorides, carbon supported transition metals and combinations of these. U.S. Publication No. 2009/0099395 discloses that HFO-1234ze may be produced via gas phase dehydrofluorination of CF₃CH₂CHF₂ by a catalytic process using a zirconium compound-carried catalyst. U.S. Publication No. 2009/0043138 discloses that HFO-1234ze and CF₃CH=CF₂ ("HFO-1225zc") may be produced from the dehydrofluorination of CF₃CH₂CHF₂ and CF₃CH₂CF₃ using oxides, fluorides, and oxyfluorides of magnesium, zinc, and mixtures of magnesium and zinc.

Methods for dehydrogenating alkanes into alkenes are known. One such method utilizes mixed metal oxides as dehydrogenation catalysts. For example, U.S. Patent No. 2,500,920 discloses that chromium oxide catalysts on an alumina support can catalyze dehydrogenation of an alkane to an alkene. Similarly, mixed metal oxide catalysts having Mo-Sb-W or Cr-Sb-W, and at least one metal selected from the group consisting of V, Nb, K, Mg, Sn, Fe, Co, and Ni, can drive oxidative dehydrogenation of propane to propene, as disclosed by U.S. Patent No. 6,239,325.

Applicants have come to recognize, however, that the above-mentioned mixed metal oxides are not suitable for use in the dehydrogenation of hydrofluorocarbons because of their tendency to react with hydrofluorocarbons, causing the conversion of metal oxides into metal oxyfluorides or even metal fluorides, and the collapse of catalyst structure.

Applicants have also come to recognize that there are significant deficiencies associated with the above-referenced methods, and that such methods are not suitable for producing HFOs such as HFO-1234yf and HFO-1234ze from certain fluorinated alkanes and alkenes. For example, Applicants have found that the dehydrofluorination of 2,3,3,3-tetrafluoropropane ("HFO-254eb") and 1,3,3,3-tetrafluoropropane ("HFO-254fb") in the presence of these catalysts forms the byproduct 3,3,3-trifluoropropene (HFO-1234zf). Because HFO-1234zf is a flammable gas, it is not preferred to have such compounds mixed with desired products such as HFO-1234yf or HFO-1234ze. Applicants have also come to recognize that HFO-1234zf has a boiling point close to HFO-1234yf and forms azeotropic or azeotrope-like compositions under certain conditions. Accordingly, separation of HFO-1234zf from HFO-1234yf is difficult and has the potential to cause yield loss during purification to HFO-1234yf.

Accordingly, Applicants have come to recognize that a need exists for a method of preparing HFOs from fluorinated alkanes, particularly for preparing HFO-1234yf and HFO-1234ze from HFC-254eb and HFC-254fb, whereby the formation of HFO-1234zf is substantially limited. This invention satisfies this need among others.

### SUMMARY OF THE INVENTION

In one aspect of the present invention, applicants have developed methods for producing fluorinated organic compounds, including HFOs in general and tetrafluoropropenes in particular embodiments, which preferably comprise converting at least one compound of formula (I):

CH₂XCHZCF₃ (I)

to at least one compound of formula (II):

CHX=CZCF₃ (II)

where X and Z are independently H or F, with the proviso that X and Z are not the same.

In certain embodiments the converting step of the present invention comprises catalytically reacting at least one compound of formula (I), preferably via dehydrogenation or oxidative dehydrogenation. The catalytic reaction step preferably comprises exposing at least one compound of formula (I) to a dehydrogenation catalyst or combination of dehydrogenation catalysts, preferably comprising one or more Group VIII noble metals supported on a metal fluoride or metal oxyfluoride support. In certain preferred embodiments, the converting step further comprises exposing at least one compound of formula (I) to one or more oxidants, preferably selected from the group consisting of O₂, CO₂, N₂O, and mixtures thereof.

In another aspect, the present invention is directed to a method of preparing fluorinated organic compounds comprising converting a reaction stream comprising at least one pentafluoropropene to a product stream comprising a mixture of at least one pentafluoropropane and a compound of formula (I), separating out at least a portion of the compound of formula (I) from the product stream, and converting the compound of formula (I) separated from the product stream to at least one compound of formula (II). In a preferred embodiment, the method further comprises substantially minimizing, and preferably substantially avoiding, the conversion of said compound of formula (I) to 3,3,3-trifluoropropene.

### DETAILED DESCRIPTION OF PREFERRED EMBODIMENTS

One aspect of the present invention relates to methods for producing HFOs from hydrofluorocarbons having at least two hydrogens on adjacent carbons via dehydrogenation or oxidative dehydrogenation. In one preferred aspect, the present invention relates to the conversion of tetrafluoropropane to tetrafluoropropene via catalytic dehydrogenation or catalytic oxidative dehydrogenation. The methods of the present invention preferably comprise converting a compound of formula (I) to an HFO, preferably a C3 HFO, more preferably a compound of formula (II). In highly preferred embodiments the present invention comprises converting HFC-254eb and/or HFC-254fb to HFO-1234yf and/or HFO-1234ze.

### CONVERSION OF COMPOUNDS OF FORMULA (I)

One beneficial aspect of the present invention is that it enables the conversion of hydrofluorocarbons, such as compounds of formula (I), to HFOs, such as compounds of formula (II), with the ability to achieve relatively high conversion and high selectivity reactions. The preferred converting step of the present invention is carried out under conditions effective to achieve conversion, and preferably conversion of at least about 15%, more preferably at least about 30%, even more preferably at least about 50%, and even more preferably about 90% of said compound of formula (I). The converting step is also capable of achieving a reaction product having at least about 50% selectivity, more preferably at least about 70% selectivity, and even more preferably at least about 90% selectivity, to compounds of formula (II). In certain embodiments a selectivity to tetrafluoropropene of at least about 95% is achievable.

The converting step can be carried out in the liquid phase or in the gas phase, or in a combination of gas and liquid phases, and it is contemplated that the reaction can be carried out batch wise, continuous, or a combination of these. Preferably, in certain embodiments, the reaction is carried out as a gas phase reaction. It is contemplated that the converting step can be carried out in any suitable reaction vessel or reactor, which may be single or multiple tubes. In preferred embodiments the converting step is carried out in a fixed-bed reactor.

The gas phase reaction may be conducted, for example, by introducing a gaseous form of a compound of formula (I), and preferably HFC-254eb, HFC-254fb, and mixtures thereof, into a suitable reaction vessel or reactor. Preferably, the vessel is comprised of materials which are resistant to corrosion, such as Hastelloy, Inconel, Monel, and/or stainless steel. In preferred embodiments, the vessel contains a catalyst, preferably a dehydrogenation or oxidative dehydrogenation catalyst, and is fitted with suitable means to heat the reaction mixture to the desired reaction temperature.

In a preferred embodiment, at least one compound of formula (I) is introduced into a reactor either in pure form, impure form, or together with an optional inert gas diluent, such as nitrogen, argon, and so on, as is readily apparent to one skilled in the art. Preferably, at least one compound of formula (I) is pre-vaporized or preheated prior to entering the reactor. Alternatively, compounds of formula (I) may be vaporized inside of the reactor.

### CATALYTIC DEHYDROGENATION

In one aspect of the present invention, the converting step is carried out via dehydrogenation in which at least one compound of formula (I) is exposed to a dehydrogenation catalyst or combination of dehydrogenation catalysts. Preferably, a feed stream comprising the compound of formula (I), optionally together with a stream of hydrogen, is fed into a dehydrogenation reactor charged with the dehydrogenation catalyst under conditions effective to produce a product stream comprising at least one compound of formula (II).

Preferably, the dehydrogenation catalyst comprises (1) one or more Group VIII noble metals supported on a metal fluoride or metal oxyfluoride support, or (2) one or more metal oxyfluoride catalysts. Non-limiting examples of Group VIII noble metals include Pt, Rh, Ru, Pd, Ir, and so forth, as are apparent to one of ordinary skill in the art. In certain embodiments the catalyst comprises the noble metal in an amount of from about 0.05 to about 10 weight %, preferably from about 0.1 to about 5 weight %, and more preferably from about 0.2 to about 1 weight %. Non-limiting examples of useful metal fluorides and metal oxyfluorides include fluorides and oxyfluorides of Ni, Co, Mg, Zr, Al, Ga, Cr, La, Y, Fe, and mixtures thereof. In certain preferred embodiments, the metal fluorides and metal oxyfluorides of the present invention are selected from the group consisting of Mg, Ni, Co, and combinations thereof. Many other catalysts may be used depending on the requirements of particular embodiments in view of the teachings contained herein. Two or more of these catalysts, or other catalysts not named here, may be used in combination.

While not wishing to be limited by theory, it is thought that certain metal fluorides and metal oxyfluorides, and in particular fluorides and oxyfluorides of Mg, Ni, and Co, will act as dehydrogenation catalysts, with limited activity as a dehydrohalogenation catalyst. Accordingly, the catalysts of the present invention are useful for the dehydrogenation of HFC-254eb and/or HFC-254fb to HFO-1234yf and/or HFO-1234ze, while limiting dehydrofluorination of HFC-254eb and/or HFC-254fb to HFO-1234zf.

While it is contemplated that a wide variety of reaction temperatures may be used, depending on relevant factors such as the catalyst being used and the most desired reaction product, it is generally preferred that the reaction temperature is from about 400°C to about 800°C. Preferred reaction temperatures may range from about 500°C to about 700°C, and more preferably from about 550°C to about 650°C.

In general, it is also contemplated that a wide variety of reaction pressures may be used, depending again on relevant factors such as the specific catalyst being used and the most desired reaction product. The reaction pressure can be, for example, super-atmospheric, atmospheric, or under vacuum, and in certain preferred embodiments is from about 0.1 to about 5 atm.

Similarly, it is contemplated that a wide variety of contact times of the reactants with the catalyst may be used depending on relevant factors such as the catalyst being used and the most desired reaction product. In certain preferred embodiments, however, the reaction time can range from about 0.5 seconds to about 120 seconds.

### CATALYTIC OXIDATIVE DEHYDROGENATION

In one aspect of the present invention, the converting step is carried out via oxidative dehydrogenation in which at least one compound of formula (I) is exposed to an oxidative dehydrogenation catalyst or combination of oxidative dehydrogenation catalysts. Preferably, a feed stream comprising the compound of formula (I) together with a stream of pure or diluted oxidant, is fed into a dehydrogenation reactor containing the oxidative dehydrogenation catalyst under conditions effective to produce a product stream comprising at least one compound of formula (II).

Preferably, the oxidative dehydrogenation catalyst comprises one or more Group VIII noble metals supported on a metal fluoride or metal oxyfluoride support, or one or more metal oxyfluoride catalysts. Non-limiting examples of such catalysts are described above, and are apparent to one of ordinary skill in the art. In certain embodiments the catalyst comprises the noble metal in an amount of from about 0.05 to about 10 weight %, preferably from about 0.1 to about 5 weight %, and more preferably from about 0.2 to about 1 weight %.

Oxidants include compounds that readily transfer oxygen atoms, or a substance that gains electrons in a redox chemical reaction. Non-limiting examples of oxidants include O₂, CO₂, and N₂O, and so forth, as are apparent to one of ordinary skill in the art. In certain embodiments, the oxidant concentration in the feedstock is in the range of from about 0.1 to 20 weight %, preferably from about 0.5 to 10 weight %, more preferably from about 1 to 5 weight %, and even more preferably from about 2 to about 3 weight %.

It is expected that many other catalysts may be used depending on the requirements of particular embodiments in view of the teachings contained herein. Of course, two or more of these catalysts, or other catalysts not named here, may be used in combination.

While it is contemplated that a wide variety of reaction temperatures may be used in catalytic oxidative dehydrogenation, depending on relevant factors such as the catalyst being used and the most desired reaction product, it is generally preferred that the reaction temperature is from about 300°C to about 700°C. Preferred reaction temperatures may range from about 400°C to about 600°C, and more preferably from about 450°C to about 550°C.

In general, it is also contemplated that a wide variety of reaction pressures may be used, depending again on relevant factors such as the specific catalyst being used and the most desired reaction product. The reaction pressure can be, for example, super-atmospheric, atmospheric, or under vacuum, and in certain preferred embodiments is from about 0.1 to about 5 atm.

Similarly, it is contemplated that a wide variety of contact times of the reactants with the catalyst may be used depending on relevant factors such as the catalyst being used and the most desired reaction product. In certain preferred embodiments, however, the reaction time can range from about 0.5 seconds to about 120 seconds.

### PREPARATION OF CATALYST

Although it is contemplated that preparation of the catalysts of the present invention may be accomplished by any means known in the art, in one embodiment the catalyst is preferably prepared by a method comprising the steps of: (a) contacting at least one metal salt, at least one solvent, and a metal fluoride or metal oxyfluoride to form a slurry; (b) removing the solvent from the slurry to form a solvent-free powder; (c) optionally calcining the powder; (d) transforming the powder into a supported catalyst; and (e) contacting the support catalyst with a gaseous composition comprising H₂ to activate the supported catalyst, wherein the activated supported catalyst comprises about 90 to about 99.5 weight % of metal fluoride or metal oxyfluoride and about 0.05 to about 10 weight percent of a zero-valent metal derived from the metal salt. In a preferred embodiment, the method comprises the steps of: (a) dissolving a salt of metal component (e.g., Pd(NO₃)₂, PdCl₂, or Pd) in a suitable solvent to form a solution; (b) adding a suitable amount of metal oxyfluoride or metal fluoride into said solution to form a slurry; (c) driving off the solvent from the slurry to form a paste; (d) drying the paste to form solvent-free powder; (e) calcining the solvent-free powder in N₂ flow for about 2 to about 8 hours at about 300 to about 500°C; (f) grinding the calcined powder to a finely divided state; (g) palletizing the fine powder into tablets; and (h) reducing the catalyst pellets in H₂ or diluted H₂ flow for about 2 to about 4 hours at about 150°C to about 250°C prior to use.

The method for preparing a metal oxyfluoride catalyst composition comprises: (a) admixing one or more of a hydroxide, an oxide, or a carbonate of a metal with the water to form a slurry; (b) substantially removing the water from the slurry to form a solid residue; (c) calcining the solid residue composition at conditions sufficient to effect substantial calcination; (d) grinding the calcined sample into fine powder, (e) pelletizing the fine powder to form pellets; and (G) contacting the pellets with HF under conditions sufficient to convert the one or more of a hydroxide, an oxide, or a carbonate of a metal to a metal oxyfluoride.

It is believed that in certain embodiments it may be possible for the catalytic dehydrogenation or catalytic oxidative dehydrogenation to proceed in the presence of a metal fluoride catalyst. The method of preparing a metal fluoride catalyst comprises: (a) adding one or more of a hydroxide, an oxide, or a carbonate of a metal to an aqueous solution of hydrogen fluoride and reacting to form a slurry of a metal fluoride; (b) substantially removing the water from the slurry to form a solid residue; (c) calcining the solid residue composition at conditions sufficient to effect substantial calcination; (d) grinding the calcined sample into fine powder; and (e) pelletizing the fine powder to form pellets of the catalyst composition.

### REGENERATION OF CATALYST

In both catalytic dehydrogenation and catalytic oxidative dehydrogenation, it is contemplated that the amount of catalyst used will vary depending on the particular parameters present in each. It is also contemplated that after use for a period of time in the converting step, the activity of the catalyst may decrease. When this occurs, the catalyst may be regenerated. Although it is contemplated that regeneration of the catalyst may be accomplished by any means known in the art, the catalyst is preferably regenerated by passing air or oxygen diluted with nitrogen over the catalyst at temperatures of from about 200°C to about 600°C, preferably from about 350°C to about 450°C, for from about 0.5 hours to about three days, optionally followed by either (1) treatment with HF at temperatures of from about 100°C to about 400°C, preferably of from about 200°C to about 350°C, for metal oxyfluoride catalysts, or (2) treatment with H₂ at temperatures of from about 100°C to about 400°C, preferably of from about 200°C to about 350°C, for Group VIII noble metal catalysts. In certain embodiments, the oxygen concentration in an oxygen/nitrogen flow used for regeneration can be in the range of from about 0.1 to about 25 weight %, preferably from about 0.5 to about 10 weight %, more preferably from about 1 to about 5 weight %, and even more preferably from about 2 to about 3 weight %.

### CONVERSION OF PENTAFLUOROPROPENES TO COMPOUNDS OF FORMULA (II) WHILE SUBSTANTIALLY LIMITING THE PRODUCTION OF 3,3,3- TRIFLUOROPROPENE

The present invention involves in one aspect methods for converting pentafluoropropenes to at least one compound of formula (II) comprising: (a) converting at least one pentafluoropropene, preferably CF₃CF=CHF ("HFO-1225ye"), in the presence of a hydrogenation catalyst to a first reaction product comprising at least one pentafluoropropane, preferably CF₃CHFCH₂F ("HFC-245eb"); and (b) converting said at least one pentafluoropropane in the presence of a dehydrofluorination catalyst to at least one compound of formula (II). Applicants have found that said first reaction product comprises compounds of formula (I) that are generated in step (a) through the hydrodefluorination of said pentafluoropropanes. Applicants have further come to recognize that notable amounts of 3,3,3-trifluoropropene (HFO-1234zf) are formed as a byproduct in step (b) through the dehydrofluorination of the at least one compound of formula (I), thereby reducing selectivity for compounds of formula (II). Accordingly, one aspect of the present invention is directed to methods of converting pentafluoropropenes, preferably HFO-1225ye, to compounds of formula (II) wherein the production of HFO-1234zf is substantially limited.

In preferred embodiments, the inventive method comprises processing a first reaction stream comprising pentafluoropropene, preferably HFO-1225ye, to a final product stream comprising at least one compound of formula (II), wherein said conditions are effective to substantially limit the concentration of HFO-1234zf present in said final product stream. Preferably, the concentration of said HFO-1234zf in said final product stream is substantially limited to a concentration of less than about 100 parts per million, preferably less than about 40 parts per million, and even more preferably less than about 20 parts per million.

In certain preferred embodiments, said processing step comprises the steps of: (a) catalytically hydrogenating said pentafluoropropene in said first reaction stream to obtain a first intermediate stream comprising pentafluoropropane, preferably HFC-245eb, and at least one compound of formula (I); and (b) separating said at least one compound of formula (I) from said first intermediate stream to obtain a separated intermediate stream and a second intermediate stream, wherein said separated intermediate stream comprises a higher concentration of said at least one compound of formula (I) than said second intermediate stream. In certain preferred embodiments, the method further comprises the steps of: (c) dehydrofluorinating said second intermediate stream under conditions effective to produce at least one compound of formula (II); and/or (d) converting said separated intermediate stream under conditions effective to produce said final product stream comprising at least one compound of formula (II).

Preferably, said catalytic hydrogenation step (a) comprises exposing said pentafluoropropene to a supported hydrogenation catalyst in the presence of hydrogen, wherein said supported hydrogenation catalyst preferably comprises a zero-valent metal disposed on a support. Non-limiting examples of suitable zero-valent metals include Pd, Ru, Pt, Rh, Ir, Fe, Co, Ni, Cu, Ag, Re, Os, Au, and combinations thereof. In certain embodiments, supported hydrogenation catalyst comprises zero-valent metal in an amount of from about 0.1 to about 10 weight %. In embodiments wherein the zero-valent metal comprises a noble metal, such as Ru, Rh, Pd, Pt, Ir, and so forth, the supported hydrogenation catalyst comprises such metals in an amount of from about 0.1 to about 5 weight %, preferably from about 0.1 to about 1 weight %. Suitable supports are characterized by being resistant to HF attack during reaction and resistant to oxidant attack during regeneration. Non-limiting examples of such supports include alpha-alumina (also known as corundum alumina), metal fluorides, and metal oxyfluorides. While it is contemplated that a wide variety of reaction temperatures may be used, depending on relevant factors such as the catalyst being used, the reaction may be conducted at a temperature of from about 50°C to about 400°C, preferably from about 100°C to about 300°C, and more preferably from about 150°C to about 250°C. While it is also contemplated that a wide variety of reaction pressures may be used, depending on relevant factors such as the catalyst being used, the reaction may be conducted at a pressures varying from atmospheric pressure, super-atmospheric pressure, or under vacuum.

Preferably, said separation step (b) comprises removing at least a portion, preferably at least about 50%, and even more preferably at least about 90% of said at least one compound of formula (I) from said first intermediate stream, preferably by distillation. In certain preferred embodiments, the distillation step is conducted in a standard distillation column at atmospheric pressure, super-atmospheric pressure, or a vacuum. Preferably, the pressure is less than about 300 psig, more preferably less than about 150 psig, and even more preferably less than 100 psig. Suitable distillation operating temperatures can be selected in view of the teachings contained herein and in view of thither operating conditions, such as the pressure of the distillation column. Further examples of such separation methods will be apparent to one skilled in the art.

Preferably, said dehydrofluorination step (c) comprises reacting said second intermediate stream with a strong caustic solution or in the presence of a dehydrofluorination catalyst. In preferred embodiments, the second intermediate stream is reacted in a caustic solution comprising KOH, NaOH, Ca(OH)₂, and CaO at an elevated temperature in a liquid phase reactor. In certain embodiments, the caustic solution is a liquid (e.g., a solution, dispersion, emulsion, or suspension, and so forth). In certain embodiments, the caustic strength of the caustic solution is from about 2 to about 100 weight %, preferably from about 5 to about 90 weight %, and more preferably from about 10 to about 80 weight %. While it is contemplated that a wide variety of reaction temperatures may be used, depending on relevant factors such as the catalyst being used, the reaction may be conducted at a temperature of from about 20°C to about 100°C, more preferably from about 40°C to about 90°C, and even more preferably from about 50°C to about 70°C. While it is contemplated that a wide variety of reaction temperatures may be used, depending on relevant factors such as the catalyst being used, the reaction may be conducted at atmospheric pressure, super-atmospheric pressure, or under vacuum. In other preferred embodiments, the second intermediate stream is reacted in the presence of a dehydrofluorination catalyst comprising fluorinated Cr₂O₃, fluorinated Al₂O₃, and/or AlF₃ at an elevated temperature in a gas phase reactor.

Preferably, said converting step (d) comprises catalytic dehydrogenation or catalytic oxidative dehydrogenation comprising the catalysts and conditions described in detail above.

### EXAMPLES

The following examples are provided for the purpose of illustrating the present invention, but without limiting the scope thereof.

### Example 1

This example illustrates the catalytic dehydrogenation of HFC-254eb. A stainless steel tube reactor (0.75" O.D. x 0.625" I.D. x 23.0" L) was charged with 20 cc of 0.5 weight % Pt/AlO_{0.75}F_{1.50} catalyst pellets. The reactor was heated by a 12" split tube furnace. A multi-point thermocouple, inserted through the catalyst bed, was used to measure the temperatures of catalyst bed. The operating conditions of a typical run included a H₂ to HFC-254eb mole ratio of 0.25, a contact time of 30 seconds, at atmospheric pressure, and a reaction temperature of 600°C. The effluent was analyzed by an on-line GC to determine the HFC-254eb conversion rate and the HFO-1234yf selectivity. After 2 hours of reaction, the HFC-254eb conversion rate and HFO-1234yf selectivity were determined to be 31% and 94%, respectively.

### Example 2

This example illustrates the catalytic oxidative dehydrogenation of HFC-254eb. A stainless steel tube reactor (0.75" O.D. x 0.625" I.D. x 23.0" L) was charged with 20 cc of 25 weight % MgO_{0.33}F_{1.33}—75 weight % of AlO_{0.75}F_{1.50} catalyst pellets. The reactor was heated by a 12" split tube furnace. A multi-point thermocouple, inserted through the catalyst bed, was used to measure the temperatures of catalyst bed and at the top of the catalyst bed. The operating conditions of a typical run included an O₂ to HFC-254eb mole ratio of 0.5, a contact time of 30 seconds, at atmospheric pressure, and a reaction temperature of 500°C. The effluent was analyzed by an on-line GC to determine the HFC-254eb conversion rate and the HFO-1234yf selectivity. After 2 hours of reaction, the HFC-254eb conversion rate and HFO-1234yf selectivity were determined to be 15% and 51%, respectively.

The present invention will now be further described by reference to the following clauses.
1. A method of preparing fluorinated organic compounds comprising converting at least one compound of formula (I):

   CH₂XCHZCF₃ (I)

   to at least one compound of formula (II):

   CHX=CZCF₃ (II)

   where X and Z are each independently H or F, with the proviso that X and Z are not the same,
   wherein the converting step comprises exposing said compound of formula (I) to a dehydrogenation catalyst or combination of dehydrogenation catalysts selected from the group consisting of one or more metal oxyfluoride catalysts, a Group VIII noble metal on a metal fluoride or metal oxyfluoride support, and combinations of these.
2. The method of clause 1, wherein said one or more Group VIII noble metals is selected from the group consisting of Pt, Rh, Ru, Pd, Ir, and combinations of these.
3. The method of clause 1, wherein said metal fluoride and said metal oxyfluoride is selected from the group consisting of fluorides and oxyfluorides of Ni, Co, Mg, Zr, Al, Ga, Cr, La, Y, Fe, and combinations of these.
4. The method of clause 1, wherein said metal fluoride and said metal oxyfluoride catalyst is selected from the group consisting of fluorides and oxyfluorides of Ni, Co, Mg, and combinations of these.
5. The method of clause 1, wherein said converting step further comprises exposing said compound of formula (I) to one or more oxidants selected from the group consisting of O₂, CO₂, N₂O, and mixtures thereof.
6. A method of converting a first reaction stream comprising at least one pentafluoropropene to a final product stream comprising at least one compound of formula (II):

   CHX=CZCF₃ (II)

   wherein X and Z are each independently H or F, with the proviso that X and Z are not the same, comprising processing said first reaction stream under conditions effective to convert said at least one pentafluoropropene to at least one compound of formula (II);
   wherein said conditions are effective to substantially limit the concentration of 3,3,3-trifluoropropene formed in said final product stream to less than about 40 parts per million; and
   wherein said processing step comprises:
      (a) reacting said at least one pentafluoropropene to obtain a first product stream comprising at least one pentafluoropropane and at least one compound of formula (I):

         CH₂XCHZCF₃ (I)

         wherein X and Z are each independently H or F, with the proviso that X and Z are not the same; and
      (b) separating said at least one compound of formula (I) from said first product stream to obtain a separated product stream and a second reaction stream, wherein said separated product stream comprises a higher weight percent of said at least one compound of formula (I) than said second reaction stream.
7. The method of clause 6 further comprising (c) reacting said second reaction stream under conditions effective to produce said at least one compound of formula (II).
8. The method of clause 6 further comprising (d) reacting said separated product stream under conditions effective to produce said at least one compound of formula (II), wherein said reacting step (d) comprises exposing said at least one compound of formula (I) to a dehydrogenation catalyst or combination of dehydrogenation catalysts selected from the group consisting of one or more metal oxyfluoride catalysts, a Group VIII noble metal on a metal fluoride or metal oxyfluoride support, and combinations of these.
9. The method of clause 8, wherein said one or more Group VIII noble metals is selected from the group consisting of Pt, Rh, Ru, Pd, Ir, and combinations of these, and wherein said metal fluoride and metal oxyfluoride is selected from the group consisting of fluorides and oxyfluorides of Ni, Co, Mg, Zr, Al, Ga, Cr, La, Y, Fe, and combinations of these.
10. The method of clause 8, wherein said reacting step (d) further comprises exposing said at least one compound of formula (I) to one or more oxidants selected from the group consisting of O₂, CO₂, N₂O, and mixtures thereof.

## Claims

1. A method of converting a first reaction stream comprising at least one pentafluoropropene to a final product stream comprising at least one compound of formula (II):
CHX=CZCF3 (II)
wherein X and Z are each independently H or F, with the proviso that X and Z are not the same, comprising processing said first reaction stream under conditions effective to convert said at least one pentafluoropropene to at least one compound of formula (II);
wherein said processing step comprises:
(a) reacting said at least one pentafluoropropene to obtain a first product stream comprising at least one pentafluoropropane and at least one compound of formula (I):
CH2XCHZCF3 (I)
wherein X and Z are each independently H or F, with the proviso that X and Z are not the same;
(b) separating said at least one compound of formula (I) from said first product stream to obtain a separated product stream and a second reaction stream, wherein said separated product stream comprises a higher weight percent of said at least one compound of formula (I) than said second reaction stream; and
(c) reacting said second reaction stream under conditions effective to produce said at least one compound of formula (II).

2. The method of claim 1, wherein step (b) comprises removing at least 50% of said at least one compound of formula (I) from said first product stream.

3. The method of claim 1 or claim 2, wherein step (b) comprises removing at least a portion of said at least one compound of formula (I) from said first product stream by distillation.

4. The method of claim 3, wherein the distillation is conducted at a pressure of less than 2.1 MPag (300 psig), preferably less than 1.0 MPag (150 psig), more preferably less than 0.69 MPag (100 psig).

5. The method of claim 3, wherein the distillation is conducted at atmospheric pressure or in a vacuum.

6. The method of any of claims 2 to 5 further comprising (d) reacting said separated product stream under conditions effective to produce said at least one compound of formula (II), wherein said reacting step (d) comprises exposing said at least one compound of formula (I) to a dehydrogenation catalyst or combination of dehydrogenation catalysts selected from the group consisting of one or more metal oxyfluoride catalysts, a Group VIII noble metal on a metal fluoride or metal oxyfluoride support, and combinations of these.

7. The method of claim 6, wherein said one or more Group VIII noble metals is selected from the group consisting of Pt, Rh, Ru, Pd, Ir, and combinations of these, and wherein said metal fluoride and metal oxyfluoride is selected from the group consisting of fluorides and oxyfluorides of Ni, Co, Mg, Zr, Al, Ga, Cr, La, Y, Fe, and combinations of these.

8. The method of claim 6 or claim 7, wherein said reacting step (d) further comprises exposing said at least one compound of formula (I) to one or more oxidants selected from the group consisting of O2, CO2, N2O, and mixtures thereof.

9. The method of any preceding claim, wherein the pentafluoropropene is CF₃CF=CHF (HFO-1225ye), the pentafluoropropane is CF₃CHFCH₂F (HFC-245eb) and the compound of formula (II) is 2,3,3,3-tetrafluoropropene (HFO-1234yf).

10. The method of claim 9, wherein the compound of formula (I) is 2,3,3,3-tetrafluoropropane (HFO-254eb).

11. The method of any preceding claim, wherein step (a) comprises catalytically hydrogenating the pentafluoropropene.

12. The method of claim 11, wherein the catalytic hydrogenation comprises reacting the pentafluoropropene with hydrogen in the presence of a metal selected from Pd, Ru, Pt, Rh, Ir, Fe, Co, Ni, Cu, Ag, Re, Os, Au, and combinations thereof, preferably wherein the catalyst is a supported catalyst comprising from 0.1 to 10 weight % of the metal, preferably wherein the catalyst support comprises alpha-alumina, metal fluorides, metal oxyfluorides and combinations thereof.

13. The method of any preceding, wherein step (c) is a dehydrofluorination comprising reacting the second intermediate stream with a strong caustic solution or in the presence of a dehydrofluorination catalyst.

14. The method of claim 13, wherein step (c) comprises a caustic dehydrofluorination reaction, preferably wherein caustic solution comprises KOH, NaOH, Ca(OH)₂, and CaO and combinations thereof, preferably wherein the reaction is conducted at a temperature of from 20°C to 100°C, more preferably from 40°C to 90°C, and even more preferably from 50°C to 70°C.

15. The method of claim 13, wherein step (c) comprises reacting the second stream in the presence of a dehydrofluorination catalyst comprising fluorinated Cr₂O₃, fluorinated Al₂O₃, and/or AlF₃ at an elevated temperature in a gas phase reactor.
